# EUROPEAN PATENT SPECIFICATION

(11) **EP 3 368 147 B1**
(45) Date of publication and mention of the grant of the patent: **04.03.2020**
(21) Application number: 16795440.3
(22) Date of filing: 27.10.2016
(51) Int. Cl.: A61N 1/32, A61N 1/30

(54) **ELECTRICAL SUBSTANCE CLEARANCE FROM THE BRAIN**
ELEKTRISCHE SUBSTANZENTFERNUNG AUS DEM GEHIRN
ÉLIMINATION DE SUBSTANCE ÉLECTRIQUE DU CERVEAU

(30) Priority: 29.10.2015 US 201514926705
(43) Date of publication of application: 05.09.2018
(73) Proprietor: Rainbow Medical Ltd., 46733 Herzliya (IL)
(72) Inventor: FOSTICK, Gideon, Givat Shmuel (IL); GROSS, Yossi, 73160 Moshav Mazor (IL); TENDLER, Alex, 34752 Haifa (IL)
(74) Representative: White, Duncan Rohan
(86) International application number: PCT/IL2016/051161
(87) International publication number: WO 2017/072769

(56) References cited:
- WO-A2-01/85027
- US-A1- 2009 131 850
- US-A1- 2014 324 128

## Description

The present disclosure relates generally to treatment and prevention of Alzheimer's disease and/or cerebral amyloid angiopathy (CAA), and specifically to electrical techniques for treating, preventing, or slowing the progression of Alzheimer's disease and/or CAA.

### BACKGROUND OF THE APPLICATION

Alzheimer's disease is a chronic neurodegenerative disease that causes dementia. Accumulation of substances such as amyloid beta and/or tau protein in the brain is widely believed to contribute to the development of Alzheimer's disease. US Patent Application Publication 2014/0324128 to Gross, which is assigned to the assignee of the present application, describes apparatus for driving fluid between first and second anatomical sites of a subject. The apparatus comprises (1) a first electrode, configured to be coupled to the first anatomical site of the subject; (2) a second electrode, configured to be coupled to the second anatomical site of the subject; and (3) a control unit, configured to (i) detect a pressure difference between the first and second anatomical sites, and (ii) in response to the detected pressure difference, drive fluid between the first and second anatomical sites by applying a treatment voltage between the first and second electrodes. Other embodiments are also described.

### SUMMARY OF THE APPLICATION

The invention provides an apparatus as claimed in claim 1.

Some embodiments of the present disclosure provide techniques for treating Alzheimer's disease and/or cerebral amyloid angiopathy (CAA). In some applications of the present invention, a parenchymal electrode is implanted in parenchyma of the brain, and a cerebrospinal fluid (CSF) electrode is implanted in a CSF-filled space of the brain, e.g., selected from a ventricular system and a subarachnoid space. Control circuitry is activated to drive the parenchymal and the CSF electrodes to clear a substance, such as amyloid beta and/or tau protein, from the brain parenchyma into the CSF-filled space of the brain.

In some applications, the techniques of the present invention, in addition to clearing the substance from the brain parenchyma into the CSF-filled space, clear the substance from the CSF-filled space to a superior sagittal sinus of the brain.

There is therefore provided, in accordance with concept 1, an apparatus comprising:
a parenchymal electrode, configured to be implanted in brain parenchyma of a subject identified as at risk of or suffering from a disease;
a cerebrospinal fluid (CSF) electrode, configured to be implanted in a CSF-filled space of a brain of the subject, the CSF-filled space selected from the group consisting of: a ventricular system and a subarachnoid space; and
control circuitry, configured to drive the parenchymal and the CSF electrodes to clear a substance from the brain parenchyma into the CSF-filled space of the brain.

There is further provided, in accordance concept 2, an apparatus comprising:
a parenchymal electrode, configured to be implanted in electrical contact with brain parenchyma of a subject identified as at risk of or suffering from a disease;
a cerebrospinal fluid (CSF) electrode, configured to be implanted in a CSF-filled space of a brain of the subject, the CSF-filled space selected from the group consisting of: a ventricular system and a subarachnoid space; and
control circuitry, configured to drive the parenchymal and the CSF electrodes to clear a substance from the brain parenchyma into the CSF-filled space of the brain.

The present invention will be more fully understood from the following detailed description of embodiments thereof, taken together with the drawings, in which:

### BRIEF DESCRIPTION OF THE DRAWINGS

Figs. 1A-C are schematic illustrations of a system for treating Alzheimer's disease, in accordance with respective applications of the present description;
Figs. 2A-B are schematic illustrations of cross-sections of a rat brain showing results of an animal experiment performed in accordance with an application of the present description;
Fig. 3 is a graph showing results of an *in vitro* experiment performed in accordance with an application of the present description; and
Figs. 4A-G are schematic illustrations of alternative configurations of the system of Figs. 1A-C, in accordance with respective applications of the present description.

### DETAILED DESCRIPTION OF APPLICATIONS

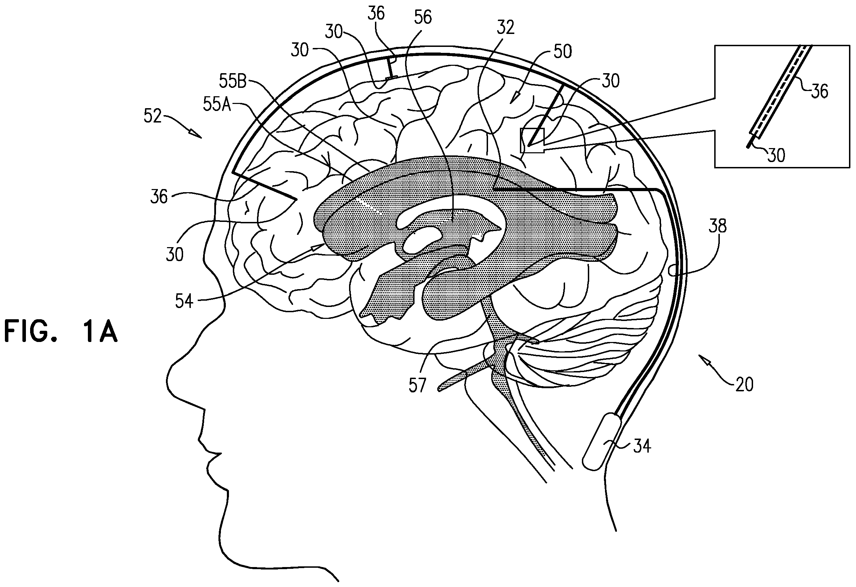

Figs. 1A-C are schematic illustrations of a system 20 for treating Alzheimer's disease and/or cerebral amyloid angiopathy (CAA), in accordance with respective applications of the present description. System 20 comprises parenchymal and cerebrospinal fluid (CSF) electrodes 30 and 32, and control circuitry 34, which is electrically coupled to parenchymal and CSF electrodes 30 and 32, typically by parenchymal and CSF electrode leads 36 and 38, respectively.

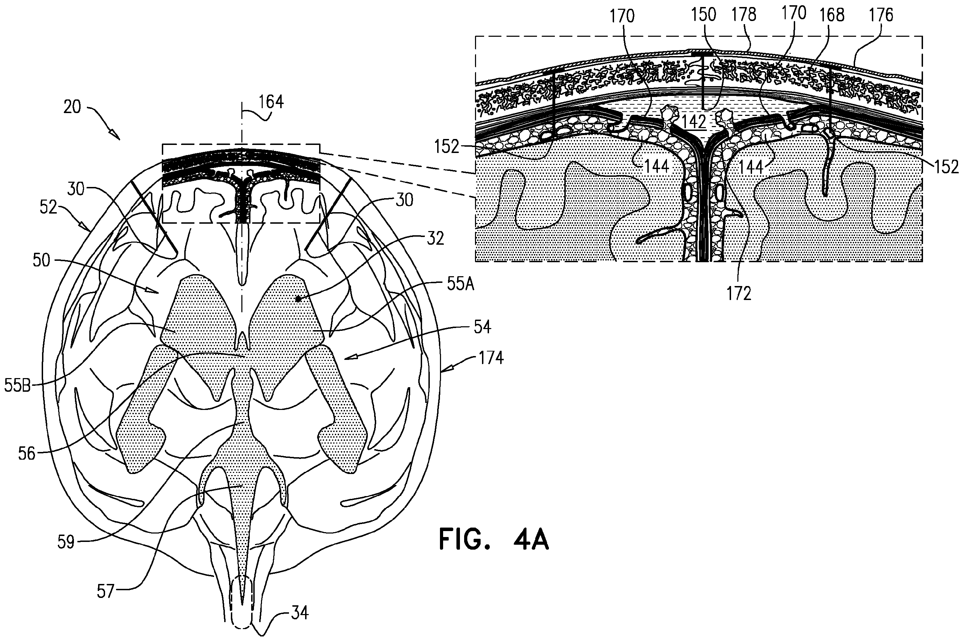

In some applications of the present description, as shown for two of parenchymal electrodes 30 illustrated in Fig. 1A, parenchymal electrode 30 is implanted in parenchyma 50 of a brain 52 of a subject identified as at risk of or suffering from Alzheimer's disease and/or from CAA, e.g., using surgical techniques similar to those used for implantation of electrodes for deep brain stimulation. Alternatively, parenchymal electrode 30 is implanted elsewhere in the subject in electrical contact with brain parenchyma 50, such as on and in contact with an outer surface of brain 52, as shown for the middle parenchymal electrode 30 illustrated in Fig. 1A. CSF electrode 32 is implanted in a CSF-filled space of the brain, such as ventricular system 54 of brain 52 or a subarachnoid space 144 (labeled in Figs. 4A-G) (e.g., cisterns of subarachnoid space 144). For example, CSF electrode 32 may be implanted using techniques known for implanting hydrocephalus shunts, *mutatis mutandis.* As used in the present application, including in the claims, ventricular system 54 includes and is limited to lateral ventricles 55 (left and right lateral ventricles 55A and 55B), a third ventricle 56, a fourth ventricle 57, a cerebral aqueduct 59 (labeled in Figs. 4A-G), interventricular foramina, a median aperture, and left and right lateral apertures.

Control circuitry 34 is activated to drive parenchymal and CSF electrodes 30 and 32 to clear a substance from brain parenchyma 50 into the CSF-filled space, such as ventricular system 54. For some applications, the substance comprises amyloid beta, metal ions, a tau protein, and/or a waste substance. As used in the present application, including in the claims, clearing a substance from the brain parenchyma is to be understood as including clearing a portion of the substance, without clearing all of the substance. Typically, in order to clear the substance, control circuitry 34 applies a voltage or a current between parenchymal and CSF electrodes 30 and 32 (i.e., control circuitry 34 regulates the voltage or the current).

Typically, a healthcare worker, such as a physician, activates control circuitry 34 to provide the functions described herein. Activating the control unit may include configuring parameters and/or functions of the control circuitry (such as using a separate programmer or external controller), or activating the control unit to perform functions preprogrammed in the control circuitry. Control circuitry 34 typically comprises appropriate memory, processor(s), and hardware running software that is configured to provide the functionality of control circuitry described herein.

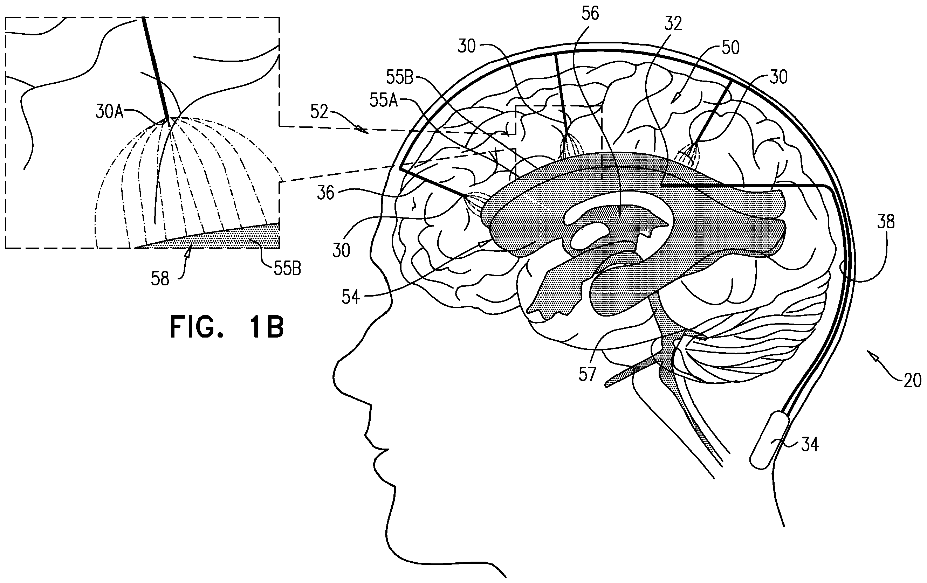

Current may flow generally through tissue that is located between parenchymal and CSF electrodes 30 and 32. Alternatively or additionally, at least a portion of the current may flow between (a) parenchymal electrode 30 and (b) an area of the CSF-filled space (e.g., ventricular system 54) nearest parenchymal electrode 30. The inventors have appreciated that because of the low electrical resistance of cerebrospinal fluid (CSF) in the CSF-filled space, such as ventricular system 54, the ventricles are to some extent a single entity electrically. Therefore, a large portion of the current flows to the nearest portion of ventricular system 54, even if CSF electrode 32 is implanted in a ventricle remote from parenchymal electrode 30. For example, as shown in Fig. 1B, if a parenchymal electrode 30A is implanted in a right hemisphere of brain 52, most of the current may flow between parenchymal electrode 30A and an area 58 of right ventricle 55B nearest parenchymal electrode 30A, even though CSF electrode 32 is implanted in left ventricle 55A.

For some applications, the voltage applied between the electrodes may clear the substance electrophoretically, because of a positive or negative charged interface between the surface of the particles of the substance and the surrounding brain tissue fluids. For these applications, the voltage applied between the electrodes causes a potential difference between brain parenchyma 50 and the CSF-filled space, such as ventricular system 54, which causes movement of the substance from brain parenchyma 50 to the CSF-filled space, such as ventricular system 54. Alternatively or additionally, for some applications, the voltage applied between the electrodes may clear the substance electroosmotically, because of a positive or negative charge of fluid in the parenchyma. For these applications, the voltage applied between the electrodes causes a potential difference between brain parenchyma 50 and the CSF-filled space, such as ventricular system 54, which causes increased flow from brain parenchyma 50 to the CSF-filled space, such as ventricular system 54, and thus increased transport of the substance from parenchyma 50 to the CSF-filled space, such as ventricular system 54.

For some applications, system 20 comprises a plurality of parenchymal electrodes 30 and/or a plurality of CSF electrodes 32. Parenchymal electrodes 30 may be implanted in one or both hemispheres of brain 52, and/or at one or more than one location in each of the hemispheres. For some applications, such as shown in Figs. 1A-C, system 20 comprises a plurality of parenchymal electrodes 30 and exactly one CSF electrode 32. For example, the single CSF electrode 32 may be implanted in one of lateral ventricles 55 or third ventricle 56, which, as discussed above, are to a large degree in good electrical connectivity with the other ventricles. For other applications (configuration not shown), system 20 comprises (a) exactly two CSF electrodes 32, which are implanted in left and right lateral ventricles 55A and 55B, respectively, or (b) exactly three CSF electrodes 32, which are implanted in left and right lateral ventricles 55A and 55B and third ventricle 56, respectively.

For applications in which system 20 comprises a plurality of parenchymal electrodes 30 and/or a plurality of CSF electrodes 32, system 20 typically comprises a corresponding plurality of parenchymal electrode leads 36 and/or a corresponding plurality of CSF electrode leads 38. Each of the leads may comprise separate electrical insulation, and/or a portion of the leads may be joined and share common electrical insulation, as shown in Figs. 1A-C for parenchymal electrode leads 36. Control circuitry 34 may be activated to independently drive parenchymal electrodes 30, e.g., using separately circuitry. Alternatively, one or more of parenchymal electrodes 30 may be shorted to one another, such that the control circuitry drives the shorted electrodes together. Control circuitry 34 may be activated to drive parenchymal electrodes 30 simultaneously or at different times.

For some applications, brain parenchyma 50 in which parenchymal electrode 30 is implanted comprises white matter of the brain.

As used in the present application, including the claims, "treating" includes both treating a subject already diagnosed with Alzheimer's disease and/or CAA (such as by delaying, slowing, or reversing progression of the disease, e.g., in a patient diagnosed at an early stage), as well as preventing the development of Alzheimer's disease and/or CAA in a subject not diagnosed with the disease and/or asymptomatic for the disease. For example, the techniques described herein may be used to prevent or delay the development of Alzheimer's disease and/or CAA in responsive to detection of an abnormal level of amyloid beta, such as using a blood test or a spinal tap.

For some applications, control circuitry 34 is configured to be implanted subcutaneously, such under skin of the skull of the subject if the housing containing the control circuitry is small, or elsewhere in the subject's body, such as in the upper chest, if the housing of the control circuitry is larger (e.g., includes batteries), with leads through the neck, or optionally in the head. For these applications, control circuitry 34 is typically driven by an external controller that is in wireless or wired communication with control circuitry 34. For some applications, the external controller is mounted on a bed of the subject (e.g., disposed within a mattress), and is configured to activate control circuitry 34 only at night, and/or only when the subject is sleeping. Such nighttime activation may to some degree mimic the natural timing of clearance of the substance (e.g., amyloid beta or tau protein) during sleep, during which the extracellular spaces are wider than during wakefulness, which allows more interstitial fluid (ISF) flow within the brain. For other applications, control circuitry 34 is configured to be disposed externally to the subject.

For some applications, control circuitry 34 is activated to drive parenchymal and CSF electrodes 30 and 32 to clear the substance by applying a non-excitatory current between parenchymal and CSF electrodes 30 and 32, i.e., the current does not cause propagation of action potentials. Thus, in these applications, control circuitry 34 is activated to set parameters of the current such that the current does not affect, or only minimally affects, neuronal activity. Alternatively, the applied current does excite brain tissue, such as to a small extent.

For some applications, control circuitry 34 is activated to drive parenchymal and CSF electrodes 30 and 32 to clear the substance by applying direct current (DC) between parenchymal and CSF electrodes 30 and 32. As used in the present application, including in the claims, direct current means a current having a constant polarity; the amplitude of the direct current may or may not vary over time, and may sometimes be zero.

For some applications, control circuitry 34 is activated to apply the direct current with an average amplitude of at least 1 mA, no more than 5 mA, and/or between 1 and 5 mA. Alternatively or additionally, for some applications, control circuitry 34 is activated to apply the direct current with an average amplitude of less than 1.2 V (such an amplitude may avoid electrolysis in the vicinity of one or both of the electrodes).

For some applications, such as when the substance is amyloid beta, control circuitry 34 is activated to configure parenchymal electrode 30 to be a cathode, and CSF electrode 32 to be an anode. Alternatively, control circuitry 34 is activated to configure parenchymal electrode 30 to be an anode, and CSF electrode 32 to be a cathode. For applications in which the voltage applied between the electrodes clears the substance electrophoretically, the selected polarity of the electrodes typically depends on whether the substance has a positive or negative effective charge. Similarly, for applications in which the voltage applied between the electrodes clears the substance electroosmotically, the selected polarity of the electrodes typically depends on whether the fluid has a positive or negative effective charge.

For some applications, control circuitry 34 is activated to apply the direct current as a series of pulses. For some applications, the series of pulses has an average pulse duration of at least 10 milliseconds, no more than 300 seconds, and/or between 10 milliseconds and 300 seconds, such as: (a) at least 10 milliseconds, no more than 100 milliseconds, and/or between 10 and 100 milliseconds, (b) at least 100 milliseconds, no more than 300 seconds (e.g., no more than 500 milliseconds), and/or between 100 and 300 seconds (e.g., between 100 and 500 milliseconds), (c) at least 500 milliseconds, no more than 5 seconds, and/or between 500 milliseconds and 5 seconds, (d) at least 5 seconds, no more than 10 seconds, and/or between 5 and 10 seconds, or (e) at least 10 seconds, no more than 100 seconds, and/or between 10 and 100 seconds. For some applications, the pulses are applied at a frequency of at least 0.001 Hz, no more than 1 kHz, and/or between 0.001 and 1 kHz, such as: (a) at least 100 Hz, no more than 1 kHz, and/or between 100 Hz and 1 kHz, (b) at least 20 Hz, no more than 100 Hz, and/or between 20 and 100 Hz, or (c) at least 1 Hz, no more than 10 Hz, and/or between 1 and 10 Hz. Alternatively or additionally, for some applications, the series of pulses has a duty cycle of at least 1%, no more than 50%, and/or between 1% and 50%, such as: (a) at least 1%, no more than 5%, and/or between 1% and 5%, (b) at least 5%, no more than 10%, and/or between 5% and 10%, (c) at least 10%, no more than 25%, and/or between 10% and 25%, or (d) at least 25%, no more than 50%, and/or between 25% and 50%. Typically, but not necessarily, the duty cycle is no more than 90%, because a given level of applied voltage produces higher current in the tissue if the capacitance in the tissue is allowed to discharge between pulses.

For some of the applications in which control circuitry 34 applies a voltage between parenchymal and CSF electrodes 30 and 32 in a series of DC pulses, the resulting current decays because of the effects of tissue electrolytes. The current may decay by about two-thirds of its initial magnitude within tens of milliseconds after commencement of application of each pulse. In order to overcome this capacitance effect, control circuitry 34 is activated to apply the voltage intermittently, in order to provide time periods between pulses during which the capacitance discharges.

For some applications, control circuitry 34 is activated to apply the voltage intermittently with a preprogrammed frequency and/or duty cycle. These parameters may be (a) applicable to all patients or a subgroup of patients, (b) set during a calibration procedure upon implantation of the electrodes, or (c) set based on a geometry of placement of parenchymal and/or CSF electrodes 30 and/or 32. Alternatively, control circuitry 34 is configured to set these parameters in real time by sensing the current resulting from the applied voltage.

For some applications, control circuitry 34 is activated to measure the current resulting from the applied voltage during each of the applied pulses, and to terminate each of the applied pulses when the magnitude of the measured current falls below a threshold value. For example, the threshold value may be a preprogrammed constant, or may be based on (e.g., a percentage of) the initial current magnitude measured upon commencement of the respective pulse. Control circuitry 34 waits during a discharge period before applying the next pulse.

For some applications, control circuitry 34 is activated to apply, between parenchymal and CSF electrodes 30 and 32, alternating current (AC) in:
- a primary subset of the pulses at a primary polarity selected to electrophoretically and/or electroosmotically clear the substance, at a primary voltage and with a primary average pulse duration, and
- a secondary subset of the pulses at a secondary polarity opposite the primary polarity, at a secondary voltage less than the primary voltage, and with a secondary average pulse duration greater than the primary average pulse duration.

Because of the lower secondary voltage, the secondary subset of the pulses to a large extent does not reverse the clearance of the substance achieved during application of the primary subset of the pulses. This technique may also help avoid electrolysis in the vicinity of one or both of the electrodes, even if the primary voltage is higher than a threshold DC voltage (e.g., 1.2 V) that might otherwise cause electrolysis.

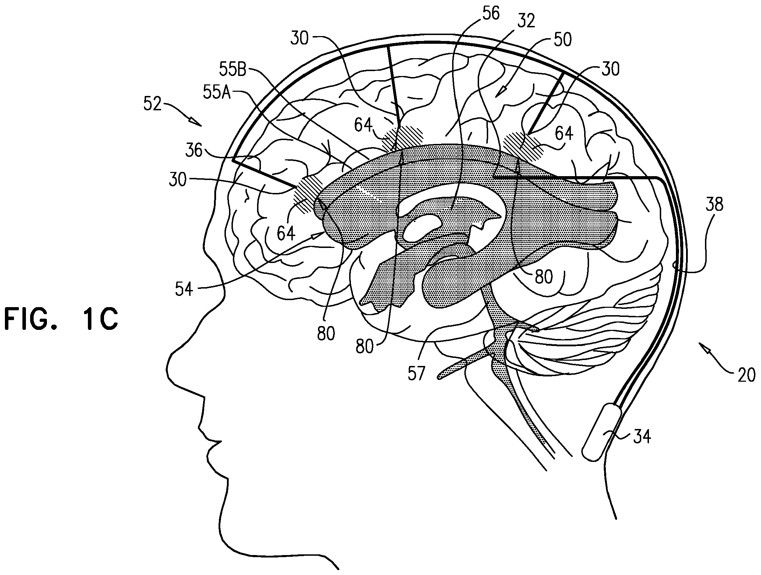

For some applications, such as illustrated in Fig. 1C, parenchymal and CSF electrodes 30 and 32 are implanted such that one or more areas of build-up 64 of the substance in brain parenchyma 50 is between the electrodes, rather than implanting parenchymal electrode 30 within the area of build-up. For example, the area(s) of build-up may include amyloid plaque and/or tau protein-related nerve tissue tangles. To this end, typically the area of build-up is first identified, for example by performing imaging of brain 52, such as MRI (e.g., functional MRI (fMRI)) or PET imaging of brain 52. As mentioned above, a plurality of parenchymal electrodes 30 and/or a plurality of CSF electrodes 32 may be implanted, such as if there is more than one area of build-up 64 of the substance.

For some applications, also such as illustrated in Fig. 1C, the one or more parenchymal electrode are implanted such that the one or more areas of build-up 64 are between parenchymal electrode 30A and respective areas 80 of the CSF-filled space, such as ventricular system 54, nearest areas of build-up 64. CSF electrode 32 may or may not be implanted near areas 80. For applications in which CSF electrode 32 is not implanted near areas 80, the substance of area of build-up 64 may still be driven into nearest areas 80 of the CSF-filled space, such as ventricular system 54, because nearest areas 80 are in fluid communication with CSF electrode 32 via CSF of the CSF-filled space, such as ventricular system 54, as discussed above. As mentioned above, a plurality of parenchymal electrodes 30 and/or a plurality of CSF electrodes 32 may be implanted, such as if there is more than one area of build-up 64 of the substance, or in general in order to provide good clearance of the substance.

For some applications, parenchymal electrode 30 is further used for applying deep brain stimulation, as is known in the art. For example, the deep brain stimulation may be applied when the electrodes are not being driven to drive the substance into the CSF-filled space, such as the ventricular system. As is known in the art, the deep brain stimulation may be applied to reduce tremor and block involuntary movements in patients with motion disorders, such as Parkinson's disease, or to treat epilepsy, cluster headaches, Tourette syndrome, chronic pain, or major depression. The implantation location of parenchymal electrode 30 may be selected to be appropriate for the treatment of a particular condition, as well as for clearing the substance.

For some applications, control circuitry 34 is activated to drive parenchymal and CSF electrodes 30 and 32 in sessions, each of which has a duration of several seconds or several minutes, or continuously for longer periods (e.g., 30 minutes). For some applications, the electrodes are not driven for a period that is at least an hour. Optionally, control circuitry 34 is activated to drive the electrodes only when the subject is sleeping, such as to take advantage of the widening of extracellular spaces and/or to inhibit any sensations that may be associated with the driving. For example, control circuitry 34 may be activated to use one or more of the electrodes as EEG electrodes to detect sleep. For some applications, power for activating and/or charging control circuitry 34 is transmitted from a wireless energy transmitter in a device applied to the head, such as a hat, or from a wireless energy transmitter in, under, or above a mattress, such as described hereinabove. For some applications, control circuitry 34 is activated to drive the electrodes according to a pre-selected schedule, such as a duty cycle, such as for a few hours per day. For example, control circuitry 34 may be configured to be controlled and/or powered by an extracorporeal control circuitry, such as a control circuitry comprising a wireless transmitter, disposed in and/or in the vicinity of the subject's bed. For some applications, one or more rest periods during which the control circuitry does not drive the electrodes are provided in the pre-selected schedule.

For any of the applications described herein, CSF electrode 32 may be implanted in one of the following sites, rather than in ventricular system 54:
- a central canal of the spinal cord (which is in fluid communication with ventricular system 54); or
- a subarachnoid space 144 (labeled in Figs. 4A-G) (which is in fluid communication with ventricular system 54 because CSF drains into cisterns of subarachnoid space 144 via foramina of ventricular system 54).

For some applications, instead of implanting CSF electrode 32 in ventricular system 54, an electrode is implanted in superior sagittal sinus 142 (labeled in Figs. 4A-G).

For any of the applications described herein, parenchymal electrode 30 may be implanted in superior sagittal sinus 142, rather than in brain parenchyma 50 (typically, in these applications, CSF electrode 32 is implanted in ventricular system 54).

Reference is again made to Figs. 1A-C. For some applications, control circuitry 34 is configured to detect a voltage difference between parenchyma 50 and the CSF-filled space, and set a level of the voltage applied between parenchymal and cerebrospinal fluid (CSF) electrodes 30 and 32 responsively to the detected voltage difference.

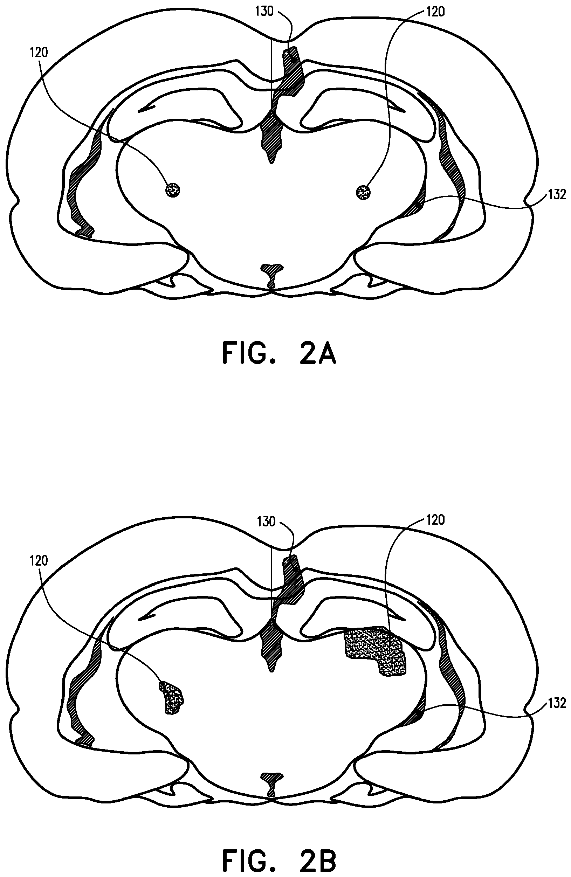

Reference is now made to Figs. 2A-B, which are schematic illustrations of cross-sections of a rat brain showing results of an animal experiment performed in accordance with an application of the present invention. A rat was anesthetized, a first electrode 130 (a piece of Pt-Ir wire soldered to a miniature connector) was inserted through a hole into the sagittal sinus, and a second electrode 132 (a pieces of Pt-Ir wire soldered to a small electronic connector) was inserted through a hole in dura mater into the right lateral ventricle.

As shown in Fig. 2A, bromephenol blue dye was stereotaxically delivered into both hemispheres of the rat brain at designated coordinates 120 and 122. By using the left hemisphere as a diffusion control, this experimental setup allowed pairwise comparisons within the same animal, thereby ruling out any other effects that might effect a directed migration of the dye in the brain.

Control circuitry was activated to apply a constant-polarity (DC) current to only the right hemisphere, between first and second electrodes 130 and 132, configuring first electrode 130 as a cathode and second electrode 132 as an anode, because bromephenol blue dye comprises effectively anionic (negatively-charged) molecules. The current was applied by repeatedly alternating between two modes: (a) a first mode, in which the current was applied continuously for 5 minutes at a magnitude of 1-2 mA, and (b) a second mode, in which the current was applied in 10-ms-duration pulses, one pulse per second (i.e., a pulse frequency of 1 Hz), at a magnitude of 1-2 mA.

Fig. 2B shows the displacement of the bromephenol blue dye after application of the current to the right hemisphere. As can be seen, the bromephenol blue dye in the left hemisphere experienced minimal dispersion and no directed displacement. In contrast, in the right hemisphere, the applied current moved the bromephenol blue dye toward the lateral ventricle. The dye moved with the average velocity of 0.28 +/- 0.006 mm/min, which was more than 14 times greater than the observed diffusion rate in the left hemisphere. In the right hemisphere, the linear displacement of the dye profile center was about 1.9±0.08 mm, while the front of the dye profile reached a maximum distance of about 2.81±0.07 mm from the center of the injection point.

The results of this experiment demonstrated that molecules of dye can be moved within brain tissue by applying a DC current using two electrodes implanted in the brain, and that in such a setup, a natural migration path is toward the ventricles. The inventors believe that application of the current between the electrodes may have moved the dye electrophoretically. The inventors also believe that implantation of the first electrode directly in brain parenchyma, rather than in the superior sagittal sinus, may provide even better current-driven movement of molecules, because the resistance of the parenchyma-sinus interface was calculated as more than two-fold higher than the resistance measured within the parenchyma, based on data collected during the experiment.

### Amyloid beta mobility and directionality assessment

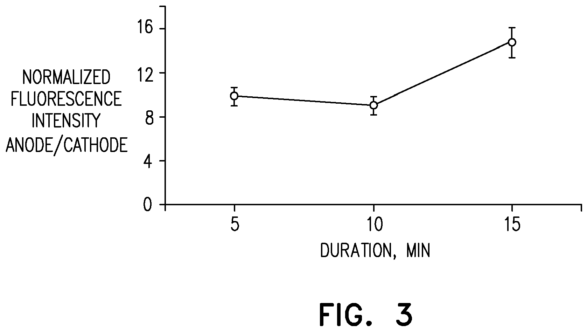

Reference is now made to Fig. 3, which is a graph showing results of an *in vitro* experiment performed in accordance with an application of the present invention. The experiment assessed the extent to which application of direct current (DC) eliminated amyloid beta peptides from an artificial cerebrospinal fluid (aCSF) solution (comprising phosphate buffered saline (PBS) solution). Pt-Ir electrodes were inserted into a compartment filled with the aCSF solution. Fluorophore-tagged amyloid beta peptides were dissolved to three different dilution levels (2:500, 5:500, and 10:500). Constant DC currents of three different durations (5, 10, and 15 minutes) were applied from a 1.5 V alkaline battery to the aCSF solution containing the fluorophore-tagged amyloid beta peptides. The directionality and overall capability of amyloid beta to undergo electrophoretic movement was assessed by densitometric analysis of fluorescence on each electrode.

The fluorescence intensity was measured at both electrodes, and the fluorescence intensity was normalized at the positively-charged electrode (anode) with respect to the negatively-charged electrode (cathode) by taking the ratio of fluorescence. Data was averaged from all the measurements and is presented as mean and standard error of mean in Fig. 3.

As can be seen in Fig. 3, current-duration-dependent enhancement of fluorescence was observed near the positively-charged electrode (anode) (for the 2:500 dilution level). The difference in fluorescence between the anodes and the cathodes was statistically significant (one tailed t-test: p<10^-15; t=12.17) for the current-duration-dependent analysis. The current-duration-dependent trend of fluorescence enhancement on the positively-charged electrode was also statistically significant for 15-minute current application vs. 5- and 10-minute current application (one-way ANOVA: p<0.001, F=8.92; Holm-Sidak post-hoc analysis: p<0.01, t=3.37 for 15 minutes vs. 5 minutes and t=3.889 for 15 minutes vs. 10 minutes due to nonspecific binding). At all concentrations there was significant attraction of the amyloid beta to the anode vs. the cathode.

These experimental results demonstrate that soluble monomeric amyloid beta in its native conformation is negatively charged in aCSF and is capable of moving in the electrical field without the need to add any amphiphilic detergents to provide the negative charge to the amyloid beta.

### Amyloid beta electrophoretic mobility assessment in wild type mouse brain parenchyma

An animal experiment was performed in accordance with an application of the present invention. 20 three-month wild-type mice were anesthetized, and soluble fluorophore-tagged amyloid beta (1-42), HiLyte™ Fluor 488-labeled, Human (AnaSpec, USA) was injected into the brain parenchyma (AP=-2, ML=0.84, DV=1.2). A first Pt-Ir electrode was implanted in brain parenchyma (AP=-2.8, ML=0.84, DV=1.5), and a second Pt-Ir electrode was implanted in the lateral ventricle (AP=-0.5, ML=0.84, DV=1.6). An electrical field generated by the current between the electrodes covered the amyloid beta injection focus. The current application was applied by the repetition of single pulses. The following parameters were used: voltage: 70 V; and frequency: 1 Hz. The current application protocol was as follows: (a) 15 minutes with a pulse duration of 1 ms; (b) 15 minutes with a pulse duration of 10 ms; and (c) 15 minutes with a pulse duration of 100 ms. The frequency was kept constant but the duty cycle was increased.

Assessment of amyloid beta movement directionality in the electrical field was conducted by using antibodies directed against 1-16 amino acid strip of 6E10 (Catalog no. SIG-39320) to visualize the traces of amyloid beta peptide movement in the electrical field. Tissue structure and cell nuclei were visualized by DAPI staining. Amyloid beta movement trajectory was evaluated at different magnifications (4x and 10x). Sagittal slices were stained with antibodies against cell nuclei (blue) and amyloid beta (6E10, green), and imaged by fluorescence microscopy.

Amyloid beta movement was visualized in mouse brains to which the electrical current was applied. The electrode inserted into the lateral ventricle was positively charged, and, similarly to the *in vitro* experiment described hereinabove with reference to Fig. 3, the applied current was capable of inducing amyloid beta movement.

These experimental results demonstrate that electrophoretic movement of amyloid beta peptides is possible in the brain parenchyma with the electrical current-application protocol used in the experiment. The directionality of amyloid beta peptide movement was similar to that observed the *in vitro* experiment described hereinabove with reference to Fig. 3.

Reference is made to Figs. 4A-G, which are schematic illustrations of alternative configurations of system 20, in accordance with respective applications of the present description. These figures show an anterior view of brain 52. In these applications, system 20 is configured to, in addition to clearing the substance (e.g., the amyloid beta, the metal ions, the tau protein, and/or the waste substance) from brain parenchyma 50 into the CSF-filled space, to clear the substance from the CSF-filled space (e.g., subarachnoid space 144) to superior sagittal sinus 142. These techniques may be used in combination with any of the techniques described hereinabove. For some of these techniques, control circuitry 34 is configured to apply the treatment current as direct current.

For some applications described with reference to Figs. 4A-G, control circuitry 34 is configured to simultaneously drive electrodes to both (a) clear the substance from brain parenchyma 50 into the CSF-filled space, and (b) clear the substance from the CSF-filled space to superior sagittal sinus 142. For example, control circuitry 34 may be configured to apply different respective voltages to parenchymal electrode 30, CSF electrode 32, and a midplane treatment electrode 150, described below. For example, control circuitry 34 may be configured to apply first, second, and third voltages to parenchymal electrode 30, CSF electrode 32, and midplane treatment electrode 150, respectively, the third voltage more positive than the second voltage, which is in turn more positive than first voltage. The total potential difference between the first and the third voltages is typically no greater than 1.2 V volt to avoid electrolysis in the vicinity of one or both of the electrodes.

For other applications described with reference to Figs. 4A-G, control circuitry 34 is configured to alternatingly drive sets of the electrodes, such as (a) during a plurality of first time periods, driving parenchymal electrode 30 and CSF electrode 32, in order to clear the substance from brain parenchyma 50 into the CSF-filled space, and (b) during a plurality of second time periods, typically not overlapping with the first time periods, driving midplane treatment electrode 150 and either CSF electrode 32 or another electrode (described below), in order to clear the substance from the CSF-filled space to superior sagittal sinus 142.

For some applications described with reference to Figs. 4A-G, control circuitry 34 is configured to clear the substance to superior sagittal sinus 142 by electroosmotically driving fluid from the CSF-filled space (e.g., subarachnoid space 144) to superior sagittal sinus 142. For some applications, control circuitry 34 is configured to drive the fluid from the CSF-filled space of the brain to superior sagittal sinus 142 by configuring midplane treatment electrode 150 as a cathode, and CSF electrode 32 as an anode.

For some applications described with reference to Figs. 4A-G, control circuitry 34 is configured to clear the substance by electrophoretically driving the substance from the CSF-filled space (e.g., subarachnoid space 144) to superior sagittal sinus 142. For some applications, application of the treatment current causes a potential difference between the CSF-filled space and superior sagittal sinus 142, which causes movement of the substance from the CSF-filled space to superior sagittal sinus 142.

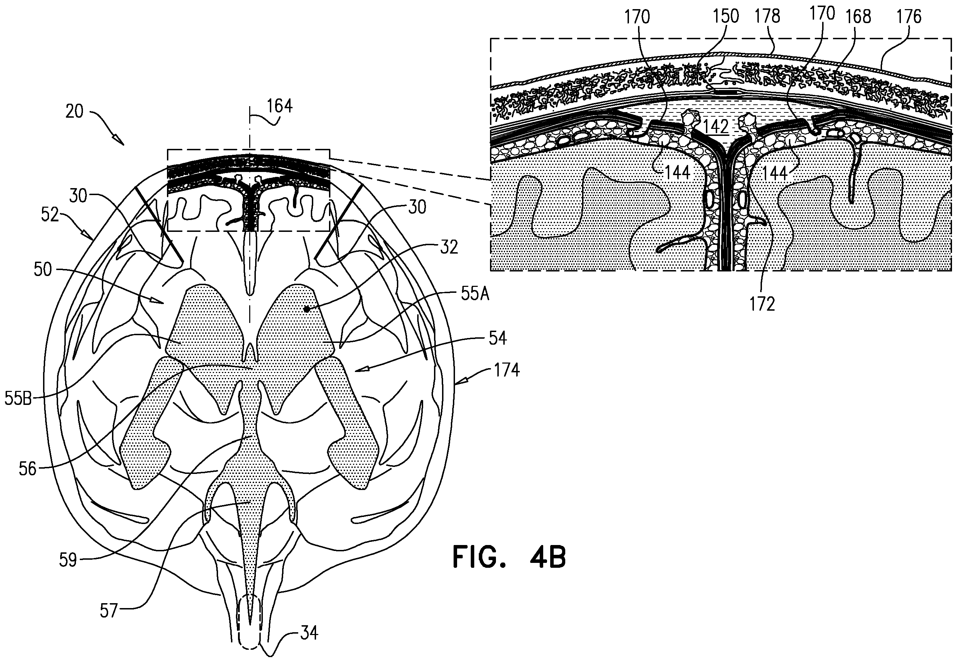

For some applications, such as shown in Fig. 4A, parenchymal electrode 30 is implanted in brain parenchyma 50, and CSF electrode 32 is implanted in the CSF-filled space, such as ventricular system 54 or subarachnoid space 144. A midplane treatment electrode 150 is disposed either (a) in superior sagittal sinus 142 (as shown in Fig. 4A), or (b) over superior sagittal sinus 142 (configuration not shown in Fig. 4A, but shown in Figs. 4B-G). A second CSF electrode 152 is implanted the CSF-filled space, such as ventricular system 54 (configuration not shown in Fig. 4A) or subarachnoid space 144 (as shown in Fig. 4A). Control circuitry 34 is activated to apply (a) a first voltage between parenchymal electrode 30 and CSF electrode 32, to clear the substance from brain parenchyma 50 into the CSF-filled space, and (b) a second voltage between midplane treatment electrode 150 and second CSF electrode 152, to clear the substance from the CSF-filled space to superior sagittal sinus 142. This technique may be used in combination with the techniques described hereinbelow with reference to Figs. 4B-G, *mutatis mutandis.*

Alternatively, for some applications, such as shown in Fig. 4B, parenchymal electrode 30 is implanted in brain parenchyma 50, and CSF electrode 32 is implanted in the CSF-filled space, such as ventricular system 54 or subarachnoid space 144. Midplane treatment electrode 150 is disposed either (a) in superior sagittal sinus 142 (as shown in Fig. 4A), or (b) over superior sagittal sinus 142 (as shown in Figs. 4B-G). Control circuitry 34 is activated to apply (a) a first voltage between parenchymal electrode 30 and CSF electrode 32, to clear the substance from brain parenchyma 50 into the CSF-filled space, and (b) a second voltage between CSF electrode 32 and midplane treatment electrode 150, to clear the substance from the CSF-filled space to superior sagittal sinus 142.

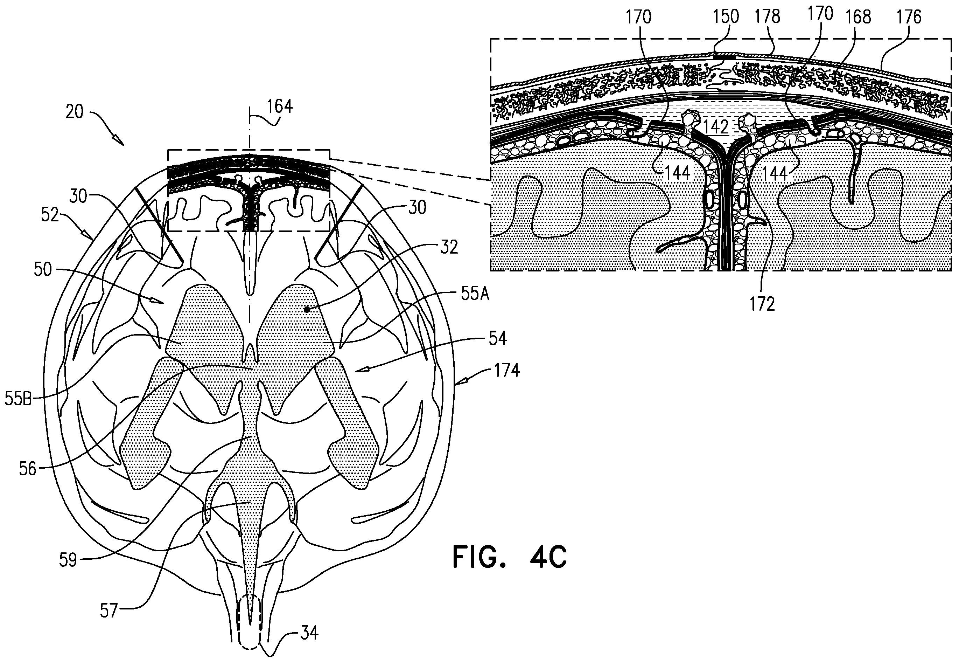

For some applications, such as shown in Figs. 4B-C, midplane treatment electrode 150 is adapted to be disposed over superior sagittal sinus 142. For some of these applications, midplane treatment electrode 150 is adapted to be disposed under a skull 168 of a head 174 of the subject, such as in contact with an outer surface of superior sagittal sinus 142 (either under the dura mater or in contact with an outer surface of the dura mater). For others of these applications, midplane treatment electrode 150 is adapted to be disposed outside and in electrical contact with skull 168. As used in the present application, including in the claims, "over the superior sagittal sinus" means aligned with the superior sagittal sinus at a location more superficial than the superior sagittal sinus, i.e., at a greater distance from a center of the head. In the configurations shown in Figs. 4B and 4C, control circuitry 34 is configured to clear the substance from the CSF-filled space to superior sagittal sinus 142, by applying a treatment current between midplane treatment electrode 150 and CSF electrode 32. Alternatively, the placements of midplane treatment electrode 150 shown in Figs. 4B and 4C are used in combination with the configuration described hereinabove with reference to Fig. 4A.

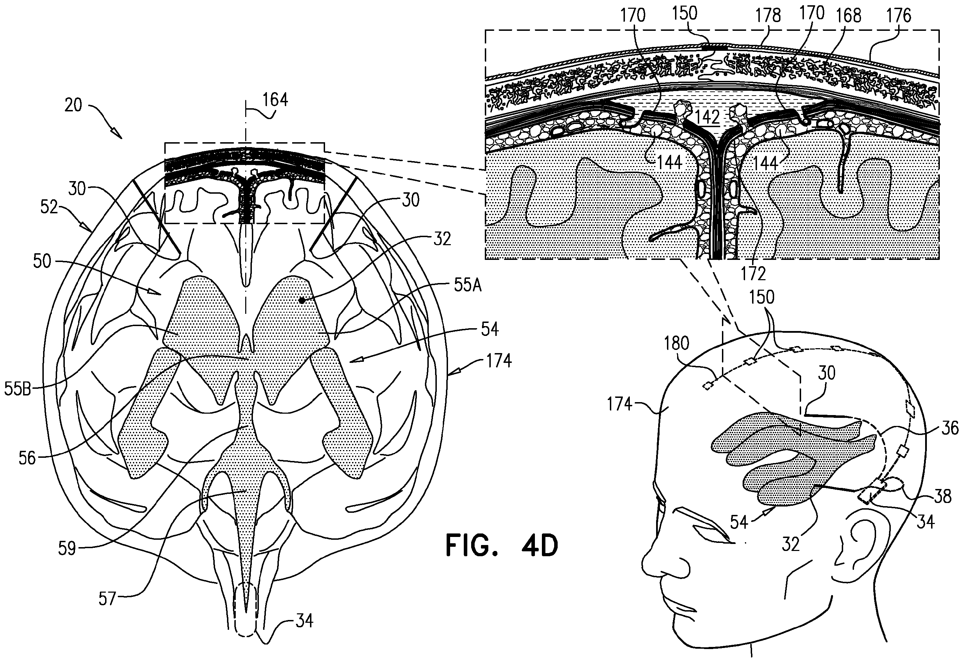

For some applications, such as shown in Fig. 4D, system 20 comprises a plurality of midplane treatment electrodes 150, such as at least 5, no more than 20, and/or between 5 and 20 midplane treatment electrodes 150. Midplane treatment electrodes 150 are disposed either (a) in superior sagittal sinus 142 (configuration not shown in Fig. 4D, but shown in Fig. 4A), or (b) over superior sagittal sinus 142 (as shown in Fig. 4D, or in Fig. 4B).

For any of the applications described herein, including, but not limited to those described with reference to Figs. 4A-G, CSF electrode 32 may be adapted to be disposed between 1 and 12 cm of a sagittal midplane 164 of skull 168. For some applications, the method may comprise implanting CSF electrode 32 between 1 and 12 cm of sagittal midplane 164 of skull 168.

For any of the applications described herein, including, but not limited to those described with reference to Figs. 4A-G, the CSF-filled space may be subarachnoid space 144, CSF electrode 32 may be a subarachnoid electrode, configured to be implanted in subarachnoid space 144, and control circuitry 34 may be configured to clear the substance from subarachnoid space 144 to superior sagittal sinus 142.

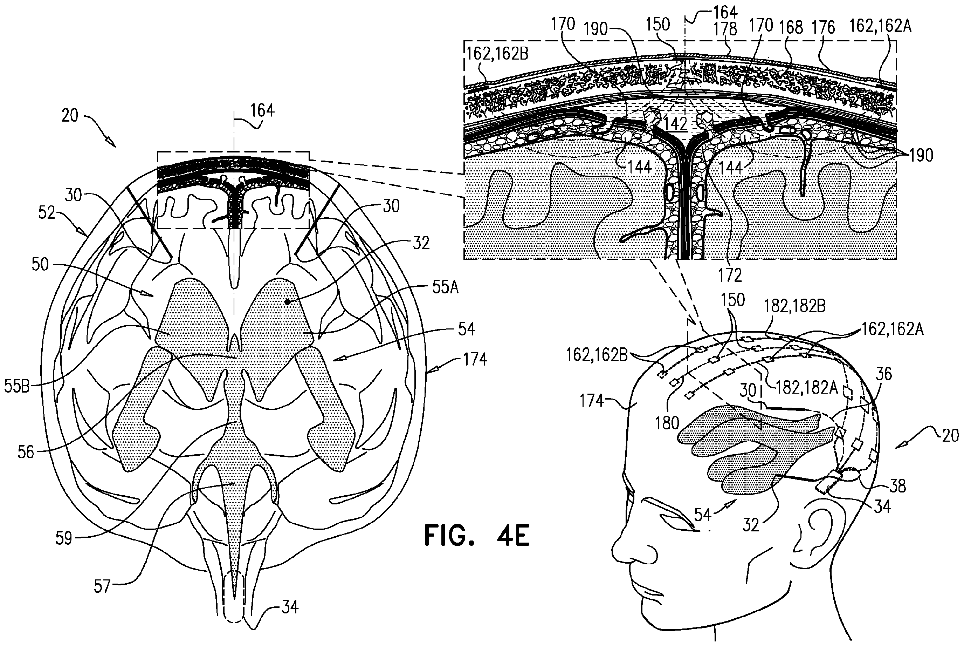

For some applications, such as shown in Fig. 4E-G, system 20 comprises (a) midplane treatment electrodes 150, adapted to be disposed over superior sagittal sinus 142, outside and in electrical contact with skull 168, and (b) lateral treatment electrodes 162, adapted to be disposed at a distance of between 1 and 12 cm of sagittal midplane 164 of skull 168 (the distance is measured in a straight line from a closest portion of each treatment electrode to sagittal midplane 164, rather than along the curvature of skull 168). Control circuitry 34 is configured to clear the substance from subarachnoid space 144 to superior sagittal sinus 142, by applying one or more treatment currents between (a) one or more of midplane treatment electrodes 150 and (b) one or more of lateral treatment electrodes 162 (each of the treatment currents is schematically illustrated in the figures by a plurality of current lines 190).

For some applications, system 20 comprises as at least 5, no more than 40, and/or between 5 and 40 lateral treatment electrodes 162, such as between 5 and 20 lateral treatment electrodes 162, or between 10 and 40 lateral treatment electrodes. For some applications, the number of each type of treatment electrode is determined based on the size of head 174 of the subject. For some applications, system 20 comprises twice as many lateral treatment electrodes 162 as midplane treatment electrodes 150.

For some applications, the one or more treatment currents applied using midplane treatment electrodes 150 and lateral treatment electrodes 162 pass between subarachnoid space 144 and superior sagittal sinus 142, via inferolateral surfaces 170 of superior sagittal sinus 142. For some of these applications, at least 40%, e.g., at least 75% or at least 90%, of the treatment currents pass between subarachnoid space 144 and superior sagittal sinus 142, via inferolateral surfaces 170 of superior sagittal sinus 142. For the applications described immediately above, the locations of midplane treatment electrodes 150 and/or lateral treatment electrodes 162 are typically selected such that the one or more treatment currents pass through inferolateral surfaces 170. For example, for configurations in which lateral treatment electrodes 162 are disposed outside and in electrical contact with skull 168, such as described with reference to Figs. 4C-G, lateral treatment electrodes 162 may be disposed at a distance of least 4 cm, no more than 12 cm, and/or between 4 and 12 cm of sagittal midplane 164 of skull 168; for configurations in which lateral treatment electrodes 162 are implanted under an arachnoid mater 172 of the subject, such as described with reference to Figs. 4C-G, lateral treatment electrodes 162 may be disposed at least 1 cm, no more than 3 cm, and/or between 1 and 3 cm of sagittal midplane 164 of skull 168.

For some applications, at least five midplane treatment electrodes 150 are disposed over superior sagittal sinus 142. Alternatively or additionally, for some applications, at least five lateral treatment electrodes 162 between 1 and 12 cm of sagittal midplane 164 of skull 168. For some applications, each of lateral treatment electrodes 162 is disposed between 1 and 12 cm of at least one of midplane treatment electrodes 150.

For some applications, midplane treatment electrodes 150 are disposed within 10 mm of sagittal midplane 164 of skull 168. Alternatively or additionally, for some applications, midplane treatment electrodes 150 are disposed such that at least one of midplane treatment electrodes 150 is at least 5 mm from another one of midplane treatment electrodes 150, no more than 20 mm from another one of midplane treatment electrodes 150, and/or between 5 and 150 mm from another one of midplane treatment electrodes 150. For some applications, at least one of lateral treatment electrodes 162 is disposed is at least 5 mm from another one of lateral treatment electrodes 162.

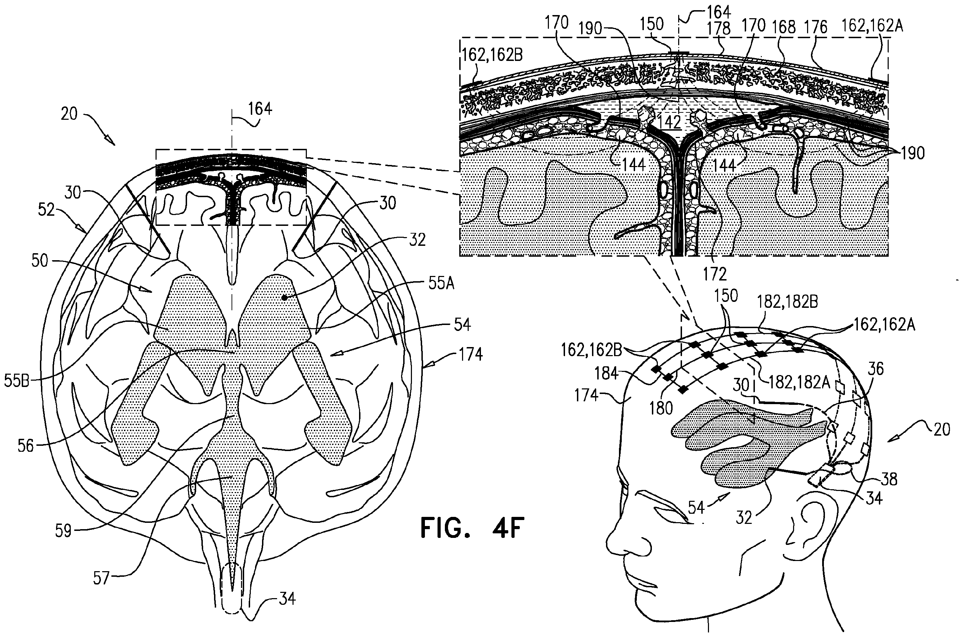

For some applications, such as shown in Fig. 4E, midplane treatment electrodes 150 are implanted under skin 176 of head 174. For other applications, such as shown in Fig. 4F, midplane treatment electrodes 150 are disposed outside head 174, such as on an external surface 178 of head 174.

For some applications, system 20 further comprises a midplane lead 180, along which midplane treatment electrodes 150 are disposed (e.g., fixed). Midplane lead 180 is disposed outside skull 168 in order to dispose midplane treatment electrodes 150 over superior sagittal sinus 142. For some applications in which midplane treatment electrodes 150 are implanted under skin 176, the implantation is performed by introducing midplane lead 180 through an incision in skin 176, typically at a posterior site of the head, and tunneling the midplane lead toward an anterior site of the head, such as near the forehead. Optionally, each of midplane treatment electrodes 150 is inserted through a respective incision in skin 176, and connected to midplane lead 180.

For some applications, such as shown in Figs. 4E-F, lateral treatment electrodes 162 are disposed outside and in electrical contact with skull 168. For some of these applications, lateral treatment electrodes 162 are implanted under skin 176 of head 174, such as shown in Fig. 4E. Alternatively, lateral treatment electrodes 162 are disposed outside head 174, such as on external surface 178 of head 174, such as shown in Fig. 4F. For some of these applications, lateral treatment electrodes 162 may be disposed at least 4 cm, no more than 12 cm, and/or between 4 and 12 cm of sagittal midplane 164 of skull 168. (As used in the present application, including in the claims, all specified ranges include their endpoints.) Such positioning may generate one or more treatment currents that pass between subarachnoid space 144 and superior sagittal sinus 142, via inferolateral surfaces 170 of superior sagittal sinus 142, as described above.

For some applications, system 20 further comprises a lateral lead 182, along which lateral treatment electrodes 162 are disposed (e.g., fixed). Lateral lead 182 is disposed outside skull 168, typically within 1 and 12 cm of sagittal midplane 164 of skull 168, in order to dispose lateral treatment electrodes 162. For some applications in which lateral treatment electrodes 162 are implanted under skin 176, the implantation is performed by introducing lateral lead 182 through an incision in skin 176, typically at a posterior site of the head, and tunneling the lateral lead toward an anterior site of the head, such as near the forehead. Optionally, each of lateral treatment electrodes 162 is inserted through a respective incision in skin 176, and connected to lateral lead 182. For some applications, instead of providing lateral lead 182, lateral treatment electrodes 162 are instead coupled to midplane lead 180. Midplane lead 180 is introduced with the lateral electrodes constrained, and, the lateral electrodes are configured upon release to extend laterally, typically automatically. This configuration may also be used for applications in which both left and right lateral electrodes are provided, as described hereinbelow.

For some applications, control circuitry 34 is activated to independently apply the treatment currents between respective pairs of midplane treatment electrodes 150 and lateral treatment electrodes 162. Such independent application of the currents allows continued effective operation of system 20 even if a low resistance should develop between the electrodes of one of the pairs (e.g., because of anatomical variations). For some of these applications, in order to enable such independent application of the currents, midplane lead 180 comprises a plurality of conductive wires corresponding to a number of midplane treatment electrodes 150, and lateral lead 182 comprises a plurality of conductive wires corresponding to a number of lateral treatment electrodes 162. Alternatively, control circuitry 34 and the electrodes implement electrical multiplexing, as is known in the art, in which case each of the leads need only comprise a single conductive wire. Alternatively, for some applications, all of midplane treatment electrodes 150 are electrically coupled to one another (such as by a single conductive wire in the midplane lead), and all of lateral treatment electrodes 162 are electrically coupled to one other (such as by a single conductive wire in the lateral lead).

For some applications of the configuration shown in Fig. 4F, system 20 further comprises one or more thin elongate support elements 184, which couple lateral leads 182 to midplane lead 180, in order to provide proper spacing and alignment between the midplane electrodes and the lateral electrodes. Support elements 184 are typically nonconductive.

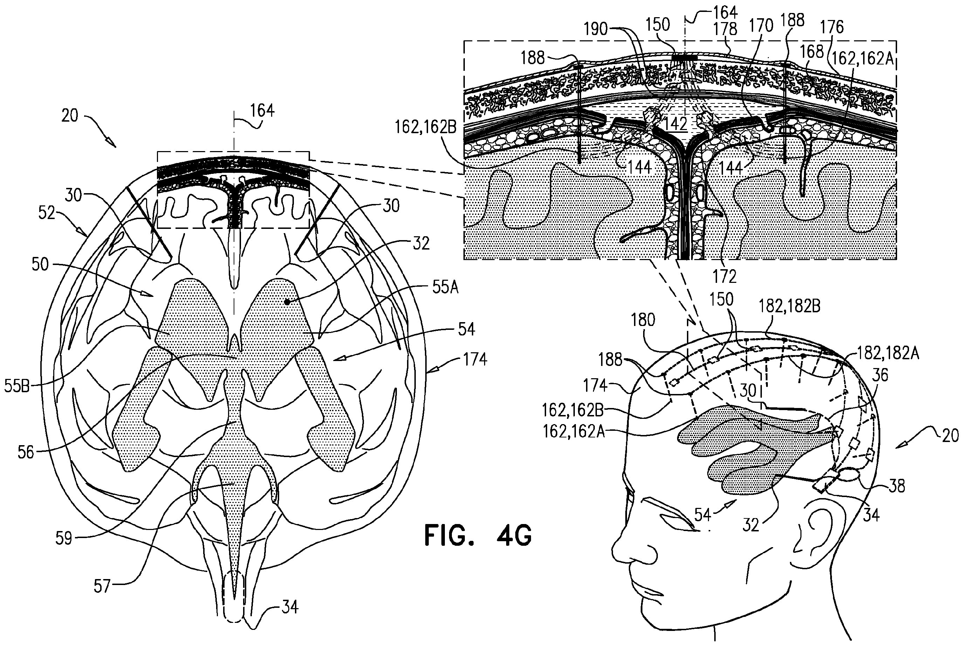

For some applications described with reference to Figs. 4A-G, control circuitry 34 is configured to apply the one or more treatment currents with an average amplitude of between 1 and 3 milliamps. (The resulting voltage is typically greater in the configuration shown in Figs. 4E-F than in the configuration shown in Fig. 4G, because the one or more treatment currents pass through skull 168 twice.)

For some applications described with reference to Figs. 4A-G, control circuitry 34 is activated to apply the one or more treatment currents as direct current, typically as a plurality of pulses, for example at greater than 500 Hz and/or less than 2 kHz, e.g., at 1 kHz. For some applications, a duty cycle of the pulses is above 90%, and for some applications pulses are not used but instead an effective duty cycle of 100% is utilized. Typically, but not necessarily, the duty cycle is 90% or lower, because a given level of applied voltage produces higher current in the tissue if the capacitance in the tissue is allowed to discharge between pulses. For other applications, control circuitry 34 is activated to apply the one or more treatment currents as alternating current with a direct current offset and a constant polarity. For example, the frequency may be at least 1 Hz, no more than 100 Hz (e.g., no more than 10 Hz), and/or between 1 Hz and 100 Hz (e.g., between 1 Hz and 10 Hz).

As mentioned above, for some applications, control circuitry 34 is configured to clear the substance by electroosmotically driving fluid from subarachnoid space 144 to superior sagittal sinus 142. For some applications, control circuitry 34 is configured to configure midplane treatment electrodes 150 as cathodes, and lateral treatment electrodes 162 as anodes. Alternatively or additionally, increased flow of cerebrospinal fluid (CSF) out of the brain's ventricular system via subarachnoid space 144, as a result of the applied voltage, may itself treat Alzheimer's disease and/or CAA, independent of any direct clearance of beta amyloid in the CSF flow.

For some applications, lateral treatment electrodes 162 comprise (a) left lateral treatment electrodes 162A, which are adapted to be disposed left of sagittal midplane 164 of skull 168, and (b) right lateral treatment electrodes 162B, which are adapted to be disposed right of sagittal midplane 164 of skull 168. For some applications, control circuitry 34 is configured to configure midplane treatment electrodes 150 as cathodes, and left and right lateral treatment electrodes 162A and 162B as left and right anodes, respectively.

As mentioned above, for some applications, control circuitry 34 is configured to clear the substance by electrophoretically driving the substance from subarachnoid space 144 to superior sagittal sinus 142. For some applications, lateral treatment electrodes 162 comprise (a) left lateral treatment electrodes 162A, which are adapted to be disposed left of sagittal midplane 164 of skull 168, and (b) right lateral treatment electrodes 162B, which are adapted to be disposed right of sagittal midplane 164 of skull 168. For some of these applications, control circuitry 34 is configured to configure the midplane treatment electrodes 150 as anodes, and left and right lateral treatment electrodes 162A and 162B as left and right cathodes, respectively. In experiments conducted on behalf of the inventor, amyloid beta was found to be attracted to the positive electrode (anode).

For some applications, lateral treatment electrodes 162 are adapted to be implanted under an arachnoid mater 172 of the subject, such as in brain parenchyma 50 (gray or white matter), as shown in Fig. 4G, or in subarachnoid space 144, such as shown in Fig. 4A. For some applications, the same electrodes serve as both parenchymal electrode 30 and lateral treatment electrodes 162, and are driven by control circuitry 34 either at the same time or at different times. For example, lateral treatment electrodes 162 may comprise needle electrodes, as is known in the art; optionally, lateral treatment electrodes 162 comprise respective proximal anchors 188. This configuration may implement any of the techniques described hereinabove with reference to Figs. 4A-F, *mutatis mutandis.*

For some of these applications, lateral treatment electrodes 162 are disposed at least 1 cm, no more than 3 cm, and/or between 1 and 3 cm of sagittal midplane 164 of skull 168. Such positioning may generate the treatment currents that pass between subarachnoid space 144 and superior sagittal sinus 142, via inferolateral surfaces 170 of superior sagittal sinus 142, as described above. For some applications, each of lateral treatment electrodes 162 is disposed between 1 and 3 cm of at least one of midplane treatment electrodes 150. For some applications, each of lateral treatment electrodes 162 is disposed between 1 and 3 cm of one of midplane treatment electrodes 150 that is closest to the lateral treatment electrode.

As mentioned above, for some applications, system 20 further comprises midplane lead 180, along which midplane treatment electrodes 150 are disposed (e.g., fixed). Midplane lead 180 is disposed outside skull 168 in order to dispose midplane treatment electrodes 150. For some of these applications, system 20 further comprises (a) a left lateral lead 182A, along which left lateral treatment electrodes 162A are disposed (e.g., fixed), and (b) a right lateral lead 182B, along which right lateral treatment electrodes 162B are disposed (e.g., fixed). Left lateral lead 186A is disposed outside skull 168, typically within 1 and 12 cm of sagittal midplane 164 of skull 168, in order to dispose left lateral treatment electrodes 162A. Right lateral lead 186B is disposed outside skull 168, typically within 1 and 12 cm of sagittal midplane 164 of skull 168, in order to dispose right lateral treatment electrodes 162B.

Reference is again made to 4A-G. For some applications, control circuitry 34 is configured to detect a voltage difference between subarachnoid space 144 and superior sagittal sinus 142, and set a level of the one or more treatment currents responsively to the detected voltage difference.

Although some of the techniques described hereinabove have been described as treating the subject by electroosmotically driving fluid from subarachnoid space 144 to superior sagittal sinus 142, the techniques may alternatively or additionally be used without electroosmosis.

The present invention is set out in the appended claims. The embodiments, aspects or examples according to the present description that do not fall within the scope of said claims are provided for illustrative purposes only and do not form part of the present invention.

## Claims

1. Apparatus comprising:
a cerebrospinal fluid (CSF) electrode (32), configured to be implanted in a CSF-filled space of a brain of a subject identified as at risk of or suffering from a disease, the CSF-filled space selected from the group consisting of: a ventricular system (54) and a subarachnoid space (144); and
control circuitry (34), **characterized in that**:
the apparatus further comprises a parenchymal electrode (30), configured to be implanted in brain parenchyma (50) of the subject, or on and in contact with an outer surface of the brain parenchyma (50), and
the control circuitry (34) is configured to drive the parenchymal and the CSF electrodes (30, 32) to clear a substance from the brain parenchyma (50) into the CSF-filled space of the brain.

2. The apparatus according to claim 1, wherein the CSF-filled space of the brain is the ventricular system (54), and wherein the CSF electrode (32) is a ventricular electrode, configured to be implanted in the ventricular system (54).

3. The apparatus according to claim 1, wherein the CSF-filled space of the brain is the subarachnoid space (144), and wherein the CSF electrode (32) is a subarachnoid electrode, configured to be implanted in the subarachnoid space (144).

4. The apparatus according to claim 1, wherein the substance includes amyloid beta or tau protein, and wherein the control circuitry (34) is configured to drive the parenchymal and the CSF electrodes (30, 32) to clear the amyloid beta or tau protein from the brain parenchyma (50) into the CSF-filled space of the brain.

5. The apparatus according to any one of claims 1, wherein the control circuitry is configured to configure the parenchymal electrode to be a cathode, and the CSF electrode to be an anode.

6. The apparatus according to claim 1, wherein the control circuitry (34) is configured to drive the parenchymal and the CSF electrodes (30, 32) to clear the substance by applying a non-excitatory current between the parenchymal and the CSF electrodes (30, 32).

7. The apparatus according to claim 1, wherein the control circuitry (34) is configured to drive the parenchymal and the CSF electrodes (30, 32) to clear the substance by applying direct current between the parenchymal and the CSF electrodes (30, 32).

8. The apparatus according to claim 7, wherein the control circuitry (34) is configured to apply the direct current with an average amplitude of between 1 and 5 mA.

9. The apparatus according to claim 7, wherein the control circuitry (34) is configured to apply the direct current with an average amplitude of less than 1.2 V.

10. The apparatus according to claim 7, wherein the control circuitry (34) is configured to apply the direct current as a series of pulses.

11. The apparatus according to claim 10, wherein the control circuitry (34) is configured to apply the direct current as the series of pulses having an average pulse duration of between 100 milliseconds and 300 seconds.

12. The apparatus according to claim 10, wherein the control unit is configured to:
drive the parenchymal and the CSF electrodes (30, 32) to clear the substance by applying a voltage between the parenchymal and the CSF electrodes (30, 32) during each of the pulses,
while applying the voltage, measure a current resulting from application of the voltage during the pulse, and
terminate the pulse upon the measured current falling below a threshold value.

13. The apparatus according to claim 1, further comprising a midplane treatment electrode (150), adapted to be disposed in or over a superior sagittal sinus (142), wherein the control circuitry (34) is configured to clear the substance from the CSF-filled space of the brain to the superior sagittal sinus (142), by applying a treatment current between the midplane treatment electrode (150) and the CSF electrode (32).

14. The apparatus according to claim 13, wherein the midplane treatment electrode (150) is adapted to be disposed over the superior sagittal sinus (142), outside and in electrical contact with a skull (168) of a head (174) of the subject.

15. The apparatus according to claim 13, wherein the midplane treatment electrode (150) is adapted to be disposed over the superior sagittal sinus (142), under a skull (168) of a head (174) of the subject.

16. The apparatus according to claim 1,
wherein the cerebrospinal fluid (CSF) electrode (32) is a first cerebrospinal fluid (CSF) electrode,
wherein the apparatus further comprises:
a midplane treatment electrode (150), adapted to be disposed in or over a superior sagittal sinus (142); and
a second cerebrospinal fluid (CSF) electrode, configured to be implanted in a CSF-filled space of a brain of the subject, the CSF-filled space selected from the group consisting of: the ventricular system (54) and the subarachnoid space (144), and wherein the control circuitry (34) is configured to clear the substance from the CSF-filled space of the brain to the superior sagittal sinus, by applying a treatment current between (a) the midplane treatment electrode (150) and (b) the second CSF electrode.

## Patentansprüche

1. Vorrichtung, umfassend:
eine Zerebrospinalflüssigkeit- bzw. CSF-Elektrode (32), konfiguriert, in einen CSF-gefüllten Raum eines Gehirns einer Person, für die das Risiko einer Erkrankung oder das Leiden daran identifiziert wurde, implantiert zu werden, wobei der CSF-gefüllte Raum aus der Gruppe ausgewählt ist, bestehend aus: einem Ventrikelsystem (54) und einem Subarachnoidalraum (144); und
Steuerschaltungen (34), **dadurch gekennzeichnet, dass**:
die Vorrichtung ferner eine Parenchymelektrode (30) umfasst, konfiguriert, im Gehirnparenchym (50) der Person oder an und in Kontakt mit einer äußeren Oberfläche des Gehirnparenchyms (50) implantiert zu werden, und
wobei die Steuerschaltungen (34) konfiguriert sind zum Ansteuern der Parenchym- und der CSF-Elektrode (30, 32), um eine Substanz aus dem Gehirnparenchym (50) in den CSF-gefüllten Raum des Gehirns zu räumen.

2. Vorrichtung nach Anspruch 1, wobei der CSF-gefüllte Raum des Gehirns das Ventrikelsystem (54) ist und wobei die CSF-Elektrode (32) eine Ventrikelelektrode ist, konfiguriert, in das Ventrikelsystem (54) implantiert zu werden.

3. Vorrichtung nach Anspruch 1, wobei der CSF-gefüllte Raum des Gehirns der Subarachnoidalraum (144) ist und wobei die CSF-Elektrode (32) eine Subarachnoidalelektrode ist, konfiguriert, in den Subarachnoidalraum (144) implantiert zu werden.

4. Vorrichtung nach Anspruch 1, wobei die Substanz Amyloid-beta- oder -tau-Protein enthält und wobei die Steuerschaltungen (34) konfiguriert sind zum Ansteuern der Parenchymelektrode und der CSF-Elektrode (30, 32), um das Amyloid-beta- oder -tau-Protein aus dem Gehirnparenchym (50) in den CSF-gefüllten Raum des Gehirns zu räumen.

5. Vorrichtung nach einem der Ansprüche 1, wobei die Steuerschaltungen konfiguriert sind, die Parenchymelektrode zu konfigurieren, eine Kathode zu sein, und die CSF-Elektrode zu konfigurieren, eine Anode zu sein.

6. Vorrichtung nach Anspruch 1, wobei die Steuerschaltungen (34) konfiguriert sind, die Parenchym- und die CSF-Elektrode (30, 32) anzusteuern, um die Substanz durch Anlegen eines nicht erregenden Stroms zwischen der Parenchym- und der CSF-Elektrode (30, 32) zu räumen.

7. Vorrichtung nach Anspruch 1, wobei die Steuerschaltungen (34) konfiguriert sind, die Parenchym- und die CSF-Elektrode (30, 32) anzusteuern, um die Substanz durch Anlegen eines Gleichstroms zwischen der Parenchym- und der CSF-Elektrode (30, 32) zu räumen.

8. Vorrichtung nach Anspruch 7, wobei die Steuerschaltungen (34) konfiguriert sind, den Gleichstrom mit einer durchschnittlichen Amplitude zwischen 1 und 5 mA anzulegen.

9. Vorrichtung nach Anspruch 7, wobei die Steuerschaltungen (34) konfiguriert sind, den Gleichstrom mit einer durchschnittlichen Amplitude von weniger als 1,2 V anzulegen.

10. Vorrichtung nach Anspruch 7, wobei die Steuerschaltungen (34) konfiguriert sind, den Gleichstrom als eine Abfolge von Impulsen anzulegen.

11. Vorrichtung nach Anspruch 10, wobei die Steuerschaltungen (34) konfiguriert sind, den Gleichstrom als eine Abfolge von Impulsen mit einer durchschnittlichen Impulsdauer zwischen 100 Millisekunden und 300 Sekunden anzulegen.

12. Vorrichtung nach Anspruch 10, wobei die Steuereinheit konfiguriert ist zum:
Ansteuern der Parenchym- und der CSF-Elektrode (30, 32), um die Substanz durch Anlegen einer Spannung zwischen der Parenchym- und der CSF-Elektrode (30, 32) während jedes der Impulse zu räumen,
während Anlegen der Spannung Messen eines aus Anlegen der Spannung resultierenden Stroms während des Impulses und
Beenden des Impulses, sobald der gemessene Strom unter einen Schwellenwert fällt.

13. Vorrichtung nach Anspruch 1, ferner umfassend eine Mittelebenen-Behandlungselektrode (150), angepasst, in oder über einem Sinus sagittalis superior (142) angeordnet zu werden, wobei die Steuerschaltungen (34) konfiguriert sind, die Substanz aus dem CSF-gefüllten Raum des Gehirns zu dem Sinus sagittalis superior (142) durch Anlegen eines Behandlungsstroms zwischen der Mittelebenen-Behandlungselektrode (150) und der CSF-Elektrode (32) zu räumen.

14. Vorrichtung nach Anspruch 13, wobei die Mittelebenen-Behandlungselektrode (150) angepasst ist, über dem Sinus sagittalis superior (142), außerhalb des und in elektrischem Kontakt mit einem Schädel (168) eines Kopfes (174) der Person angeordnet zu werden.

15. Vorrichtung nach Anspruch 13, wobei die Mittelebenen-Behandlungselektrode (150) angepasst ist, über dem Sinus sagittalis superior (142), unter einem Schädel (168) eines Kopfes (174) der Person angeordnet zu werden.

16. Vorrichtung nach Anspruch 1,
wobei die Zerebrospinalflüssigkeit- bzw. CSF-Elektrode (32) eine erste Zerebrospinalflüssigkeit- bzw. CSF-Elektrode ist,
wobei die Vorrichtung ferner umfasst:
eine Mittelebenen-Behandlungselektrode (150), angepasst, in oder über einem Sinus sagittalis superior (142) angeordnet zu werden; und
eine zweite Zerebrospinalflüssigkeit- bzw. CSF-Elektrode, konfiguriert, in einem CSF-gefüllten Raum eines Gehirns der Person implantiert zu werden, wobei der CSF-gefüllte Raum aus der Gruppe ausgewählt ist, bestehend aus: dem Ventrikelsystem (54) und dem Subarachnoidalraum (144), und wobei die Steuerschaltungen (34) konfiguriert sind, die Substanz aus dem CSF-gefüllten Raum des Gehirns zu dem Sinus sagittalis superior durch Anlegen eines Behandlungsstroms zwischen (a) der Mittelebenen-Behandlungselektrode (150) und (b) der zweiten CSF-Elektrode zu räumen.

## Revendications

1. Appareil comprenant :
une électrode de fluide cérébrospinal (CSF) (32), qui est configurée de manière à ce qu'elle soit implantée à l'intérieur d'un espace rempli de CSF d'un cerveau d'un sujet qui est identifié comme étant sous risque de souffrir d'une maladie ou comme souffrant d'une maladie, l'espace rempli de CSF étant sélectionné parmi le groupe qui est constitué par : un système ventriculaire (54) et un espace sous-arachnoïdien (144) ; et
un circuit de commande (34) ; **caractérisé en ce que** :
l'appareil comprend en outre une électrode de parenchyme (30), qui est configurée de manière à ce qu'elle soit implantée à l'intérieur d'un parenchyme cérébral (50) du sujet, ou sur et en contact avec une surface externe du parenchyme cérébral (50); et
le circuit de commande (34) est configuré de manière à ce qu'il pilote les électrodes de parenchyme et de CSF (30, 32) de manière à ce qu'elles éliminent une substance depuis le parenchyme cérébral (50) à l'intérieur de l'espace rempli de CSF du cerveau.

2. Appareil selon la revendication 1, dans lequel l'espace rempli de CSF du cerveau est le système ventriculaire (54), et dans lequel l'électrode de CSF (32) est une électrode ventriculaire, qui est configurée de manière à ce qu'elle soit implantée à l'intérieur du système ventriculaire (54).

3. Appareil selon la revendication 1, dans lequel l'espace rempli de CSF du cerveau est l'espace sous-arachnoïdien (144), et dans lequel l'électrode de CSF (32) est une électrode sous-arachnoïdienne, qui est configurée de manière à ce qu'elle soit implantée à l'intérieur de l'espace sous-arachnoïdien (144).

4. Appareil selon la revendication 1, dans lequel la substance inclut une protéine bêta amyloïde ou une protéine tau, et dans lequel le circuit de commande (34) est configuré de manière à ce qu'il pilote les électrodes de parenchyme et de CSF (30, 32) de manière à ce qu'elles éliminent la protéine bêta amyloïde ou la protéine tau depuis le parenchyme cérébral (50) à l'intérieur de l'espace rempli de CSF du cerveau.

5. Appareil selon la revendication 1, dans lequel le circuit de commande est configuré de manière à ce qu'il configure l'électrode de parenchyme de telle sorte qu'elle soit une cathode et l'électrode de CSF de telle sorte qu'elle soit une anode.

6. Appareil selon la revendication 1, dans lequel le circuit de commande (34) est configuré de manière à ce qu'il pilote les électrodes de parenchyme et de CSF (30, 32) de manière à ce qu'elles éliminent la substance en appliquant un courant de non excitation entre les électrodes de parenchyme et de CSF (30, 32).

7. Appareil selon la revendication 1, dans lequel le circuit de commande (34) est configuré de manière à ce qu'il pilote les électrodes de parenchyme et de CSF (30, 32) de manière à ce qu'elles éliminent la substance en appliquant un courant continu entre les électrodes de parenchyme et de CSF (30, 32).

8. Appareil selon la revendication 7, dans lequel le circuit de commande (34) est configuré de manière à ce qu'il applique le courant continu selon une amplitude moyenne qui se situe entre 1 et 5 mA.

9. Appareil selon la revendication 7, dans lequel le circuit de commande (34) est configuré de manière à ce qu'il applique le courant continu selon une amplitude moyenne qui est inférieure à 1,2 V.

10. Appareil selon la revendication 7, dans lequel le circuit de commande (34) est configuré de manière à ce qu'il applique le courant continu en tant que série d'impulsions.

11. Appareil selon la revendication 10, dans lequel le circuit de commande (34) est configuré de manière à ce qu'il applique le courant continu en tant que série d'impulsions qui présentent une durée d'impulsion moyenne qui se situe entre 100 millisecondes et 300 secondes.

12. Appareil selon la revendication 10, dans lequel l'unité de commande est configurée de manière à ce qu'elle réalise les actions qui suivent :
le pilotage des électrodes de parenchyme et de CSF (30, 32) de manière à ce qu'elles éliminent la substance en appliquant une tension entre les électrodes de parenchyme et de CSF (30, 32) pendant chacune des impulsions ;
pendant l'application de la tension, la mesure d'un courant qui résulte de l'application de la tension pendant l'impulsion ; et
l'arrêt de l'impulsion suite au fait que le courant mesuré chute en deçà d'une valeur de seuil.

13. Appareil selon la revendication 1, comprenant en outre une électrode de traitement de plan médian (150), qui est adaptée de manière à ce qu'elle soit disposée à l'intérieur ou au-dessus d'un sinus sagittal supérieur (142), dans lequel le circuit de commande (34) est configuré de manière à ce qu'il élimine la substance depuis l'espace rempli de CSF du cerveau jusqu'au sinus sagittal supérieur (142), en appliquant un courant de traitement entre l'électrode de traitement de plan médian (150) et l'électrode de CSF (32).

14. Appareil selon la revendication 13, dans lequel l'électrode de traitement de plan médian (150) est adaptée de manière à ce qu'elle soit disposée au-dessus du sinus sagittal supérieur (142), à l'extérieur d'un crâne (168) d'une tête (174) du sujet et en contact électrique avec celui-ci.

15. Appareil selon la revendication 13, dans lequel l'électrode de traitement de plan médian (150) est adaptée de manière à ce qu'elle soit disposée au-dessus du sinus sagittal supérieur (142), au-dessous d'un crâne (168) d'une tête (174) du sujet.

16. Appareil selon la revendication 1, dans lequel :
l'électrode de fluide cérébrospinal (CSF) (32) est une première électrode de fluide cérébrospinal (CSF) ; dans lequel :
l'appareil comprend en outre :
une électrode de traitement de plan médian (150), qui est adaptée de manière à ce qu'elle soit disposée à l'intérieur d'un sinus sagittal supérieur (142) ou au-dessus de ce même sinus sagittal supérieur ; et
une seconde électrode de fluide cérébrospinal (CSF), qui est configurée de manière à ce qu'elle soit implantée à l'intérieur d'un espace rempli de CSF d'un cerveau du sujet, l'espace rempli de CSF étant sélectionné parmi le groupe qui est constitué par : le système ventriculaire (54) et l'espace sous-arachnoïdien (144), et dans lequel le circuit de commande (34) est configuré de manière à ce qu'il élimine la substance depuis l'espace rempli de CSF du cerveau jusqu'au sinus sagittal supérieur, en appliquant un courant de traitement entre (a) l'électrode de traitement de plan médian (150) et (b) la seconde électrode de CSF.
